# EUROPEAN PATENT SPECIFICATION

(11) **EP 3 049 053 B1**
(45) Date of publication and mention of the grant of the patent: **24.05.2017**
(21) Application number: 14758943.6
(22) Date of filing: 04.09.2014
(51) Int. Cl.: A61K 8/49, A61Q 5/00, A61Q 19/02, A61K 8/97, A61K 36/28

(54) **A COMPOSITION FOR SKIN AND SCALP HEALTH**
ZUSAMMENSETZUNG FÜR DIE HAUT- UND KOPFHAUTGESUNDHEIT
COMPOSITION POUR LA PEAU ET LA SANTÉ DU CUIR CHEVELU

(30) Priority: 26.09.2013 EP 13186093
(43) Date of publication of application: 03.08.2016
(73) Proprietor: Unilever NV, 3013 AL Rotterdam (NL); Unilever PLC, a company registered in England and Wales under company no. 41424, London EC4Y 0DY (GB)
(72) Inventor: DAMODARAN, Anita, Bangalore 560 066 (IN); SANYAL, Aryasekhar, West Bengal 734404 (IN)
(74) Representative: Fijnvandraat, Arnoldus
(86) International application number: PCT/EP2014/068808
(87) International publication number: WO 2015/043907

(56) References cited:
- JP-A- 2010 013 400
- L. P. NIKONOVA ET AL: "Granilin - A new lactone from Inula grandis", CHEMISTRY OF NATURAL COMPOUNDS, vol. 8, no. 3, May 1972 (1972-05), pages 286-289, XP055096221, ISSN: 0009-3130, DOI: 10.1007/BF00563730
- DATABASE CA [Online] CHEMICAL ABSTRACTS SERVICE, COLUMBUS, OHIO, US; 1977, MARUYAMA, MASAO ET AL: "Granilin as a bactericide and fungicide", XP002718670, retrieved from STN Database accession no. 1977:528879 cited in the application & JP 52 034921 A (MITSUI TOATSU CHEMICALS, INC., JAPAN) 17 March 1977 (1977-03-17)

## Description

### Field of the Invention

The present invention relates to a skin lightening composition. The invention more specifically relates to a composition and a method for lightening skin using a specific molecule extractable from a natural material which additionally has been found to have anti-dandruff properties.

### Background of the invention

Highly pleasing skin appearance is one of the most desired expectations from personal care products from most consumers around the world. In tropical countries where consumers generally have dark skin, there is a desire to have lighter skin appearance. In consumers who live far from the tropical countries e.g. the Caucasian people who generally have lighter skin, there is a need among such consumers to have an even tanned tone of their skin. Any exposure of the skin to sunlight, in such consumers often leads to blotchy skin, referred to as freckles and in some cases they experience hyperpigmentation in localized areas of the skin. Most consumers experience blemishes on their skin after exposure to sun, on healing of wounds or after drying up of acne. Most people, all over the world, notice darker skin on their underarms (i.e in their axilia) as compared to other regions of their skin, although the underarms are rarely exposed to the sun. They prefer an even skin tone in their axilia as compared to other parts of the body. In all of the above cases, consumers rely on cosmetic solutions to their skin appearance problems.

Thus, smooth, soft and glowing skin with even skin tone and colour is desired by all consumers who use personal care compositions for their skin. To ensure that their skin continues to be even and does not get tanned unevenly, one commonly used approach is to include sunscreens or sunblocks in such personal care compositions. Another approach to controlling the colour, tone and appearance of the skin is the skin lightening approach where chemicals are added to personal care compositions which alter the formation of melanin in the skin through biochemical transformation in the stratum corneum thereby changing the colour and appearance of the skin. This approach is capable of lightening the skin beyond the basic colour of skin. While this approach has been used successfully in many cosmetic products, manufacturers are still struggling to convince more and more people to use such products. One of the impediments is that many consumers do not like using synthetic actives on their skin and prefer using products which have a natural origin.

Dandruff is another problem experienced by many people. Dandruff is a condition where dry flakes fall off from the scalp and produce an unpleasant appearance when they fall on hair or on the shoulders. The exact etiology of dandruff formation is not fully understood. However, a microorganism *Malassezia sp.* is believed to be responsible in some way for dandruff formation. Various anti-dandruff actives incorporated in products like shampoo have been developed and are commercially available. Similar to skin lightening, more and more people are looking for hair and scalp care products of natural origin.

The present inventors have found that the molecule granilin is capable of providing skin lightening benefits and additionally anti-dandruff benefits. The molecule granilin can be extracted and concentred from *Inula grandis.*

*"Granilin* as a *bactericide and fungicide",* an article published in Jpn. Kokai Tokkyo Koho (1977), by Maruyama, Masao; Haino, Hiroyuki; Morikawa, Osamu; Tachibana, Hajime, discloses that this compound is extracted from *Carpesium abrotanoides* and is found to be an effective bactericide and fungicide e.g. when a dilute solution is sprayed on rice that had been inoculated with Xanthomonas oryzae it decreased the infection on the leaves by about 75%.

JP 2010013400 (Ichimaru Pharcos Inc) discloses a melanin generation inhibitor useful in cosmetics for providing whitening effect and for preventing or improving liver spots, freckles and dry skin, and comprises an extract of *Inula racemosa* or *Andrographis paniculata* as active ingredient.

Granilin as a synthetic active or as an extract from *Inula grandis* is heretofore not known for use in skin lightening or for anti-dandruff benefits.

It is therefore an object of the present invention to obviate at least some drawbacks of the prior art and provide a skin lightening composition comprising actives from natural sources.

It is another object of the present invention to provide an active extractable from natural sources for incorporation in hair and scalp care compositions for anti-dandruff benefits.

### Summary of the Invention

The present invention relates to a composition for care of skin and scalp comprising
(a) 0.0001 to 10% by weight of a compound of formula 1; and 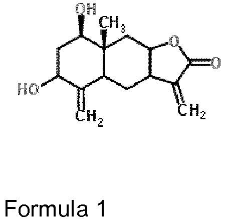
(b) a cosmetically acceptable base.

According to another aspect of the invention, there is provided a method of lightening skin comprising the step of applying a composition of the invention on to a desired skin surface.

According to yet another aspect of the invention there is provided a method of treating dandruff comprising the step of applying a composition of the invention on to the desired scalp surface.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

"Skin lightening Composition" as used herein, is meant to include a composition for topical application to skin of mammals, especially humans. Such a composition may be generally classified as leave-on or rinse off, and includes any product applied to a human body primarily for lightening skin but may also improve appearance, cleansing, odor control or general aesthetics. The composition is preferably a leave-on composition. The composition of the present invention can be in the form of a liquid, lotion, cream, foam, scrub, gel, soap bar or toner, or applied with an implement or via a face mask, pad or patch. Preferred forms of the composition are liquid, lotion, cream, foam, or gel. Non-limiting examples of the compositions include leave-on skin lotions and creams, shampoos, conditioners, shower gels, toilet bars, antiperspirants, deodorants, depilatories, lipsticks, foundations, mascara, sunless tanners and sunscreen lotions. "Skin" as used herein is meant to include skin on the face and body (e.g., neck, chest, back, arms, underarms, hands, legs, and buttocks).

The invention provides for a skin lightening composition comprising granilin which is represented by the chemical formula given below.

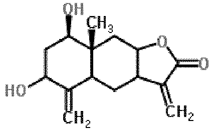

Granilin is a compound of the sesquiterpene lactone class. Granilin is present in 0.0001 to 10%, preferably in 0.001 to 2%, by weight of the composition.

Granilin is preferably used in the invention as an extract of *Inula grandis.*

Inula is a large genus of about 90 species of flowering plants in the family Asteraceae, native to Europe, Asia and Africa. *Inula grandis* found in the Almalyk region of Republic of Uzbekistan have been used to isolate Granulin.

It is preferred that the aerial parts of the plant collected during stem formation period of *Inula grandis* is used for preparing the extract for use in the composition of the invention.

A preferred process for the extraction of granilin from *Inula grandis* is given below. The dried comminuted raw material was treated with methanol, the extract was concentrated in vacuum to small volume, and it was extracted successively with gasoline, carbon tetrachloride, and chloroform. The chloroform extract was evaporated and passed through a column filled with Kapron and alumina (activity grade IV). When the latter sorbent was washed with benzene and the eluate was concentrated, large colorless crystals deposited with melting point of 197-198°C (from methanol).

The composition of the invention comprises a cosmetically acceptable base. The cosmetically acceptable base is preferably a powder, bar, liquid, lotion, cream, foam, or gel more preferably a cream, lotion, gel or emulsion.

Personal care compositions may be prepared using different cosmetically acceptable emulsifying or non-emulsifying systems and vehicles. Preferred cosmetically acceptable bases comprise 1 to 25% fatty acid. A further preferred aspect provides for inclusion of 0.1 to 10% soap. A highly suitable base is a cream. Vanishing creams are especially preferred. Vanishing cream bases generally comprise 5 to 25% w/w fatty acid and 0.1 to 10% w/w soap. Vanishing cream base gives a highly appreciated matty feel to the skin. C₁₂ to C₂₀ fatty acids are especially preferred in vanishing cream bases, further more preferred being C₁₄ to C₁₈ fatty acids. The most preferred fatty acid is stearic acid. The fatty acid may also be a mixture of palmitic and stearic acid. The fatty acid in the composition is more preferably present in an amount in the range of 5 to 20% w/w of the composition. Soaps in the vanishing cream base include alkali metal salt of fatty acids, like sodium or potassium salts, most preferred being potassium stearate. The soap in the vanishing cream base is generally present in an amount in the range of 0.1 to 10%, more preferably 0.1 to 3% w/w of the composition. Generally the vanishing cream base in cosmetic compositions is prepared by taking a desired amount of total fatty matter and mixing with potassium hydroxide in desired amounts. The soap is usually formed in-situ during the mixing. The personal care composition when formulated as a vanishing cream preferably comprises 60 to 85%, more preferably 65 to 80% w/w water. Water is generally present in many compositions prepared as per the invention and is generally present in 40 to 85% by weight of the composition.

The composition of the invention may comprise another skin lightening agent other than the active of the present invention. This additional skin lightening agent is preferably chosen from a vitamin B3 compound or its derivative e.g. niacin, nicotinic acid, niacinamide or any other well known skin lightening agent. Most preferred additional skin lightening agent is niacinamide. Niacinamide, when used, is preferably present in an amount in the range of 0.1 to 10%, more preferably 0.2 to 5% w/w of the composition.

The personal care composition may preferably additionally comprise one or more UV sunscreens. The UV sunscreens may be inorganic or organic. A wide variety of organic sunscreen agents are suitable for use in combination with the essential ingredients of this invention. Most suitable organic sunscreen are 2-ethylhexyl-p-methoxycinnamate (as a UVB sunscreen agent) and/ or butylmethoxydibenzoylmethane (as a UVA sunscreen agent).

A safe and effective amount of sunscreen may be used in the compositions of the present invention. The composition preferably comprises from about 0.1% to about 10%, more preferably from about 0.1% to about 5% w/w of a sunscreen agent.

Useful inorganic sun-blocks are also preferably used in the present invention. These include, for example, zinc oxide iron oxide, silica, such as fumed silica, and titanium dioxide.

Ultrafine titanium dioxide in either of its two forms, namely water-dispersible titanium dioxide and oil-dispersible titanium dioxide is especially suitable for the invention. Water-dispersible titanium dioxide is ultra-fine titanium dioxide, the particles of which are non-coated or which are coated with a material to impart a hydrophilic surface property to the particles. Examples of such materials include aluminium oxide and aluminium silicate.

Oil-dispersible titanium dioxide is ultrafine titanium dioxide, the particles of which exhibit a hydrophobic surface property, and which, for this purpose, can be coated with metal soaps such as aluminium stearate, aluminium laurate or zinc stearate, or with organosilicone compounds.

By "ultrafine titanium dioxide" is meant particles of titanium dioxide having an average particle size of less than 100 nm, preferably 70 nm or less, more preferably from 10 to 40 nm and most preferably from 15 to 25 nm. The total amount of sun block that is preferably incorporated in the composition according to the invention is from 0.1 to 5% w/w of the composition.

The composition according to the invention may also comprise other diluents. The diluents act as a dispersant or carrier for other materials present in the composition, so as to facilitate their distribution when the composition is applied to the skin.

Diluents other than water can include liquid or solid emollients, solvents, humectants, thickeners and powders.

The cosmetically acceptable base is usually from 10 to 99.9%, preferably from 50 to 99% w/w of the composition, and can, in the absence of other cosmetic adjuncts, form the balance of the composition. The composition of the invention may comprise water.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The composition is formulated in any known format, more preferred formats being creams or lotions.

The composition of the present invention may be formulated as a product for treating dandruff. Preferred composition for such use include hair gels, creams, hair oils as leave-on compositions and shampoo and conditioner as wash-off compositions. Of these, wash off compositions are preferably a shampoo composition.

The composition for providing the anti-dandruff benefit is preferably a shampoo composition. Shampoos compositions are generally aqueous, i.e. they have water or an aqueous solution as their major component. Suitably, the composition will comprise from 50 % to 98%, preferably from 60 % to 90% water by weight based on the total weight of the composition.

The shampoo composition generally comprises one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Preferred anionic cleansing surfactants are sodium lauryl sulphate, sodium lauryl ether sulphate(n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 % to 45%, preferably from 1.5 % to 35%, more preferably from 5 % to 20 % by total weight anionic cleansing surfactant based on the total weight of the composition.

Optionally shampoo compositions may contain further ingredients as described below to enhance performance and/or consumer acceptability.

Shampoo compositions may include co-surfactants, to help impart aesthetic, physical or cleansing properties to the composition. An example of a co-surfactant is a nonionic surfactant, which can be included in an amount ranging from 0.5 % to 8%, preferably from 2 % to 5% by weight based on the total weight of the composition. Representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco mono-isopropanolamide.

Another preferred example of a co-surfactant is an amphoteric or zwitterionic surfactant, which can be included in an amount ranging from 0.5% to about 8%, preferably from 1% to 4% by weight based on the total weight of the composition. Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocoamidopropyl betaine.

The total amount of surfactant (including any co-surfactant, and/or any emulsifier) in a shampoo composition of the invention is generally from 1% to 50%, preferably from 2 % to 40%, more preferably from 10 % to 25% by total weight surfactant based on the total weight of the composition.

Cationic polymers are preferred ingredients in a shampoo composition. A particularly suitable type of cationic polysaccharide polymer that can be used is a cationic guar gum derivative, such a guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162. Cationic polymer will generally be present in a shampoo composition of the invention at levels of from 0.01 % to 5%, preferably from 0.05 % to 1 %, more preferably from 0.08 % to 0.5% by total weight of cationic polymer based on the total weight of the composition.

Preferably, an aqueous shampoo composition further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Lubrizol.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition at levels of from 0.1 % to 10%, preferably from 0.5 % to 6%, more preferably from 0.9 % to 4% by total weight of suspending agent based on the total weight of the composition.

Another aspect of the present invention provides for a method of lightening skin comprising the step of applying a composition of the invention on to a desired skin surface.

Yet another aspect of the present invention provides for a method of treating dandruff comprising the step of applying a composition of the invention on to the desired scalp surface.

According to yet another aspect of the invention there is provided use of a composition of the invention for providing skin lightening benefits. According to yet another aspect of the present invention there is provided use of a composition of the invention for providing anti-dandruff benefits. Also claimed is Granilin for use in the treatment of dandruff. Further, the present invention also claims compositions of the present invention for use in the treatment of dandruff. The use is preferably non-therapeutic.

Granilin for use in carrying out the Examples illustrated in this specification were sourced from Timtec, Newark, Delaware, USA.

The invention is now further described by way of the following non-limiting examples.

### Examples

### Examples A and 1 to 3:

The following compositions were used to determine reduction in melanin content.
Example A: Ethyl resorcinol at 0.00014% was used.
Example 1: Granilin at 0.0005 % was used.
Example 2: Granilin at 0.00075% was used.
Example 3: Granilin at 0.001% was used.

The skin lightening potential was measured using reduction in melanin content as the invitro assay.

10⁴ cells of Neonatal Human Epidermal Melanocytes (NHEM) and 10⁴ cells of HaCat were seeded per well of a NUNC 12 well plate and incubated at 37°C in a humidified incubator with 5% CO2 atmosphere for 24 hours. Melanocyte Growth Medium (MGM) and Keratinocyte Growth Medium (KGM) were mixed in a ratio of 1:1 and 1 ml of this mixed media was used to seed the cells per well of 12 well plate. The media were also supplemented with 10 U/ml penicillin G, 0.1 mg/ml streptomycin sulphate. NEHM between passages 4-6 and HaCats between passage 56- 70 were used for experimentation. The cells were tested to be Mycoplasma-free. 24 hours later, fresh media containing actives was added. It was ensured that appropriate vehicle controls were included in the assay (Final concentrations of DMSO was ≤ 0.1% in both the treatments and the vehicle control). The treated cells were incubated for a further 72 hours at 37°C in a humidified incubator with 5% CO₂. At the end of the time point the plates were proceded for calcein viability assay follower by melanin content assay (MCA).

Calcein-Am was procured from Fluka, product code 14210P03. Cell viability was evaluated in all plates using the Calcein-AM Viability assay. At the time of harvest, the spent media from all wells of the 12 well plate was aspirated and rinsed once with 400 µl of 1xPBS-Ca-Mg, including wells without cells (Blank wells). 1µM of Calcein-AM (working stock) was prepared in 1xPBS-Ca-Mg from 1mM stock (e.g. 10µl of 1mM Calcein stock in 100% DMSO diluted in 10ml of 1xPBS-Ca-Mg). 400µl of above Calcein-AM working stock solution was added to each of the active treated and control wells. The plate was covered with aluminium foil, to protect from light and incubated for 30 minutes in a CO₂ incubator. After 30 minutes of dye incubation, fluorescence was measured using TECAN infinite M1000 instrument (excitation at 490nm and emission at 520nm). After fluorescence measurement, the plates were rinsed once with regular 1X PBS and then proceed for MCA.

After the calcein assay, 150 µl of MCA reagent (10% DMSO in 1 N NaOH) was added to each well of MCA plate (12 well Nunc plate) and incubated for 1 hour at 60°C in a rocker incubator. 120 µl of solution from each well were transferred into a well of a 384 well plate. The absorbance was read in a multi well plate reader at 405 nm.

For the non-cytotoxic concentrations (based on calcein viability calculations), MCA as %control was calculated and reported.

The reduction in melanin content is summarised in Table -1 below.

**Table -1**

| Example | Active | Concentration, wt% | Reduction in melanin content |
|---|---|---|---|
| A | Ethyl resorcinol | 0.00014 | 21.5% |
| 1 | Granilin | 0.0005 | 30.4% |
| 2 | Granilin | 0.00075 | 47.6% |
| 3 | Granilin | 0.001 | 52.3% |

The data in Table -1 above indicates that granilin is indicated through reduction in melanin content assay to have excellent skin lightening ability, better than a very good positive control ethyl resorcinol.

## Claims

1. A composition for care of skin and scalp comprising
(a) 0.0001 to 10% by weight of a compound of formula 1; and 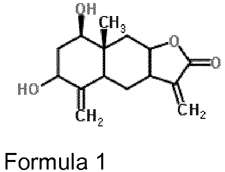
(b) a cosmetically acceptable base.

2. A composition as claimed in claim 1 comprising 0.001 to 2% compound of formula 1 by weight of the composition.

3. A composition as claimed in claim 1 or claim 2 wherein said compound of formula 1 is extracted and concentrated from *Inula grandis.*

4. A composition as claimed in any one of the preceding claims wherein said cosmetically acceptable base is in the form of powder, bar, liquid, lotion, cream, foam, or gel.

5. A method of lightening skin comprising the step of applying a composition as claimed in any one of the preceding claims on to a desired skin surface.

6. A non-therapeutic method of treating dandruff comprising the step of applying a composition as claimed in any one of the preceding claims 1 to 4 on to the desired scalp surface.

7. Use of a composition as claimed in any one of the preceding claims 1 to 4 for providing skin lightening benefits.

8. Composition as claimed in any one of the preceding claims 1 to 4 for use in the treatment of dandruff.

## Patentansprüche

1. Zusammensetzung zur Pflege von Haut und Kopfhaut, umfassend
(a) 0,0001 bis 10 Gewichts-% einer Verbindung der Formel 1 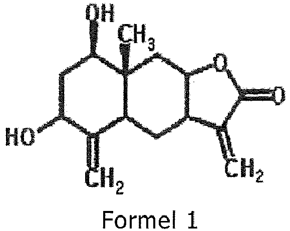 und
(b) einen kosmetisch annehmbaren Grundbestandteil.

2. Zusammensetzung, wie im Anspruch 1 beansprucht, umfassend 0,001 bis 2% der Verbindung der Formel 1, bezogen auf das Gewicht der Zusammensetzung.

3. Zusammensetzung, wie im Anspruch 1 oder Anspruch 2 beansprucht, wobei die Verbindung der Formel 1 aus *Inula grandis* extrahiert und konzentriert wird.

4. Zusammensetzung, wie in irgendeinem der vorhergehenden Ansprüche beansprucht, wobei der kosmetisch annehmbare Grundbestandteil in Form eines Pulvers, Stabes, einer Flüssigkeit, Lotion, Creme, eines Schaums oder Gels vorliegt.

5. Verfahren zum Aufhellen von Haut, umfassend den Schritt des Aufbringens einer Zusammensetzung, wie in irgendeinem der vorhergehenden Ansprüche beansprucht, auf eine gewünschte Hautoberfläche.

6. Nicht therapeutisches Verfahren zur Behandlung von Schuppen, umfassend den Schritt des Aufbringens einer Zusammensetzung, wie in irgendeinem der vorhergehenden Ansprüche 1 bis 4 beansprucht, auf die gewünschte Kopfhautoberfläche.

7. Verwendung einer Zusammensetzung, wie in irgendeinem der vorhergehenden Ansprüche 1 bis 4 beansprucht, zum Bereitstellen von Hautaufhellungsvorteilen.

8. Zusammensetzung, wie in irgendeinem der vorhergehenden Ansprüche 1 bis 4 beansprucht, zur Verwendung bei der Behandlung von Schuppen.

## Revendications

1. Composition pour le soin de la peau et du cuir chevelu comprenant
(a) de 0,0001 à 10 % en masse d'un composé de formule 1 ; et 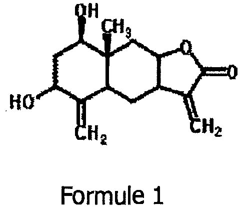
(b) une base cosmétiquement acceptable.

2. Composition selon la revendication 1 comprenant de 0,001 à 2 % de composé de formule 1 en masse de la composition.

3. Composition selon la revendication 1 ou la revendication 2, dans laquelle ledit composé de formule (1) est extrait et concentré à partir de *Inula grandis*

4. Composition selon l'une quelconque des revendications précédentes, dans laquelle ladite base cosmétiquement acceptable est dans la forme de poudre, de barre, de liquide, de lotion, de crème, de mousse, ou de gel.

5. Procédé d'éclaircissement de la peau comprenant l'étape d'application d'une composition selon l'une quelconque des revendications précédentes sur une surface de peau souhaitée.

6. Procédé non thérapeutique de traitement de pellicules comprenant l'étape d'application d'une composition selon l'une quelconque des revendications 1 à 4 précédentes sur la surface de cuir chevelu souhaitée.

7. Utilisation d'une composition selon l'une quelconque des revendications 1 à 4 précédentes pour fournir des bénéfices d'éclaircissement de la peau.

8. Composition selon l'une quelconque des revendications 1 à 4 précédentes pour une utilisation dans le traitement de pellicules.
